# EUROPÄISCHE PATENTSCHRIFT

(11) **EP 0 589 314 B1**
(45) Veröffentlichungstag und Bekanntmachung des Hinweises auf die Patenterteilung: **22.01.1997**
(21) Anmeldenummer: 93114621.1
(22) Anmeldetag: 11.09.1993
(51) Int. Cl.: C07C 29/149, C07D 307/06

(54) **Verfahren zur Herstellung von 2-Methyl-1,4-butandiol und 3-Methyltetrahydrofuran**
Process for the preparation of 2-methyl-1,4-butanediol and 3-methyltetrahydrofuran
Procédé de préparation de 2-méthyl-1,4-butanediol et 3-méthyltétrahydrofurane

(30) Priorität: 23.09.1992 DE 4231782
(43) Veröffentlichungstag der Anmeldung: 30.03.1994
(73) Patentinhaber: BASF Aktiengesellschaft, 67063 Ludwigshafen (DE)
(72) Erfinder: Weyer, Hans-Juergen, Dr., D-6800 Mannheim 1 (DE); Fischer, Rolf, Dr., D-6900 Heidelberg (DE); Merger, Franz, Dr., D-6710 Frankenthal (DE); Frank, Juergen, Dr., D-6703 Limburgerhof (DE); Henkelmann, Jochem, Dr., D-6800 Mannheim 1 (DE); Siegel, Hardo, Dr., D-6720 Speyer (DE); Ruehl, Thomas, Dr., D-6710 Frankenthal (DE)

(56) Entgegenhaltungen:
- EP-A- 0 276 012
- EP-A- 0 277 562
- EP-A- 0 304 696
- WO-A-86/03189
- WO-A-88/00937
- DE-A- 3 801 863
- US-A- 3 859 369
- US-A- 3 956 318
- US-A- 3 975 449
- US-A- 4 124 600

## Beschreibung

Die vorliegende Erfindung betrifft ein neues Verfahren zur Herstellung von 2-Methyl-1,4-butandiol und 3-Methyltetrahydrofuran (3-Methyl-THF).

Diese Produkte sind bisher durch verschiedene Verfahren zugänglich.

Nach der Lehre der EP-A 277 562 führt die Hydrierung von Citronensäure an Pd oder Re auf TiO₂, ZrO₂ oder Kohlenstoff zu 3-Methyl-THF und 3- und 4-Methylbutyrolacton. Die Selektivität von 70 % für 3-Methyl-THF läßt allerdings noch Wünsche offen, wenn eine großtechnische Ausübung des Verfahrens angestrebt wird.

Die US-A 3 956 318 lehrt die Umsetzung von alkylsubstituierten ω-Hydroxyepoxiden zu 3-Methyl-THF mit Wasserstoff bei 50 bis 250° und 0,7 bis 350 bar an Katalysatoren, die ein Metall der Gruppen 8 bis 10 des Periodensystems enthalten. Bei 20 bis 200°C kann aus diesen Ausgangsverbindungen unter sonst gleichen Bedingungen nach der US-A 3 975 449 2-Methyl-1,4-butandiol hergestellt werden. Beide Verfahren sind wenig attraktiv, da die Herstellung der Ausgangsverbindungen aufwendig ist.

In der JP-A 49/9463, der JP-A 49/9464 und in der JP-A 50/1038 wird die Herstellung von u.a. Methyl-γ-butyrolacton und/oder 3-Methyl-THF durch Hydrierung von Methylmalein- oder Methylfumarsäure beschrieben. Auch in diesen Fällen ist die Herstellung der Ausgangsmaterialien aufwendig. Die Verfahren sind weiterhin durch technisch aufwendige Maßnahmen (Temperatursteuerung bzw. eine bestimmte Anordnung des Katalysators) geprägt.

Die Hydroformylierung von But-2-en-1,4-diol, Abtrennung des Katalysators sowie die Hydrierung der Reaktionsmischung führt nach der Lehre der US-A 3 859 369 zu 2-Methyl-1,4-butandiol, welches durch saure Katalyse zu 3-Methyl-THF umgesetzt werden kann. Die Hydroformulierung der innenliegenden Doppelbindung gelingt jedoch nur in geringer Ausbeute, Hauptprodukt ist immer 1,4-Butandiol. Chirales 2-Methyl-1,4- butandiol kann aus chiralem 2-Methylbernsteinsäurediester durch katalytische Hydrierung hergestellt werden (DE-A 3 801 863).

Gemäß der Lehre der EP-A 276 012 wird Itaconsäure an Pd/Re auf TiO₂ zu 3- bzw. 4-Methyl-γ-butyro-lacton sowie zu Spuren an 2-Methyl-1,4-butandiol hydriert.

Es stellte sich die Aufgabe, ein Verfahren zur Herstellung von 2-Methyl-1,4-butandiol und 3-Methyl-THF bereitzustellen, das die genannten Nachteile nicht aufweist.

Demgemäß wurde ein Verfahren zur Herstellung von 2-Methyl-1,4-butandiol und 3-Methyl-THF gefunden, das dadurch gekennzeichnet ist, daß man Verbindungen der allgemeinen Formeln I und II 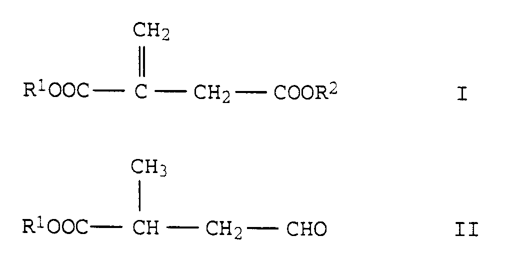 in denen R¹ und R² für Wasserstoff oder eine C₁-C₈-Alkylgruppe stehen und die Formylgruppe von II auch als Acetal mit C₁-C₈-Alkoholen vorliegen kann, in Gegenwart von Kupfer oder von Kupferverbindungen bei einem Druck von 50 bis 300 bar mit Wasserstoff umsetzt.

Bei den Ausgangsverbindungen I handelt es sich um Itaconsäure (R¹ und R² stehen für Wasserstoff) oder deren C₁-C₈-Alkylmonoester- oder- diester. Die Alkylgruppen sind z.B. Methyl, Ethyl, n-Propyl, iso-Propyl, n-Butyl und iso-Butyl. Bevorzugt wird aber Itaconsäure selbst, die man sehr vorteilhaft durch fermentativen Zuckerabbau herstellen kann (z.B. US-A 3 044 941).

Die Ausgangsverbindungen II leiten sich von der 3-Formyl-2-methyl-propionsäure ab. Die Alkylreste sind Reste wie Ethyl, n-Propyl, n-Butyl und iso-Butyl, vorzugsweise aber Methyl. 3-Formyl-2-methyl-propionsäuremethylester kann in guter Ausbeute und Selektivität durch Hydroformylierung von Methyl-methacrylat hergestellt werden (Bull. Chem. Soc. Japan 50 (1977) 2351). Die Aldehydfunktion kann auch in Form eines Acetals mit C₁-C₈-Alkanolen vorliegen.

Die Katalysatoren enthalten als wesentliche Bestandteile Kupfer oder eine Kupferverbindung. Da die Katalysatoren in der Regel mit Wasserstoff aktiviert werden, liegen die aktiven Komponenten überwiegend in metallischer Form vor.

Die Katalysatoren können weitere Bestandteile enthalten. Es sind hier z.B. Zink, Chrom, Molybdän, Wolfram und Mangan zu nennen, aber auch saure Verbindungen wie Mineralsäuren, z. B. Schwefelsäure, Phosphorsäure und Chlorwasserstoffsäure, Lewissäuren wie Bortrifluorid und Zinkchlorid und Heteropolysäuren wie Wolframatophosphorsäure.

Die Katalysatoren können in kompakter Form, d.h. ohne Träger, vorzugsweise aber als Trägerkatalysatoren eingesetzt werden. Die Art des Trägermaterials ist in der Regel nicht kritisch. Es können daher übliche Trägermaterialien wie Siliciumdioxid, Aluminiumdioxid, Titandioxid, Zirkoniumdioxid, Aktivkohle, Silicate und Zeolithe verwendet werden.

Erforderlichenfalls können zur Herstellung der Katalysatoren Bindemittel oder Formhilfsmittel eingesetzt werden.

Die Herstellung der Katalysatoren erfolgt nach an sich bekannten Methoden, etwa indem man Carbonate und Hydroxyde der aktiven Metalle und der Trägerverbindungen gemeinsam fällt, formt, calciniert und mit Wasserstoff aktiviert.

Die Trägerkatalysatoren können beliebige Gestalt haben, z.B. Splitt, Kugeln, Zylinder, Stränge oder Ringe.

Die Reaktion kann mit oder ohne Lösungsmittel durchgeführt werden. Geeignete Lösungsmittel sind z.B. Ether wie Tetrahydrofuran, Alkohole, besonders C₁-C₈-Alkylalkohole wie Methanol, Ethanol und iso-Propanol, und Wasser sowie Mischungen dieser Lösungsmittel.

Die Reaktionstemperaturen betragen vorzugsweise 100 bis 350°C, besonders bevorzugt 150 bis 250°C. Der Druck beträgt 50 bis 300 bar. Die Reaktion kann in der Gas- oder Flüssigphase diskoninuierlich und kontinuierlich durchgeführt werden. Bei fest angeordneten Katalysatoren kann die Sumpf- oder Rieselfahrweise gewählt werden. Weiterhin ist es möglich, suspendierte Katalysatoren zu verwenden.

Es haben sich Katalysatorbelastungen von 0,01 bis 1, vorzugsweise 0,05 bis 0,3 kg Ausgangsmaterial je Kilogramm Katalysator und Stunde bewährt.

Als Reaktoren können z.B. Rührkessel, Rohrreaktoren oder Rohrbündelreaktoren verwendet werden.

Das anfallende Reaktionsgemisch kann in bekannter Weise, vorzugsweise destillativ, getrennt werden.

Das erfindungsgemäße Verfahren erlaubt die einfache Herstellung von 2-Methyl-1,4-butandiol und 3-Methyl-THF aus gut zugänglichen Ausgangsmaterialien.

Es hat sich herausgestellt, daß eine hohe Reaktionstemperatur, eine niedrige Katalysatorbelastung und der Einsatz von Katalysatoren, die eine saure Komponente enthalten, in der Regel zu einer verstärkten Bildung von 3-Methyl-THF führen.

2-Methyl-1,4-butandiol ist eine wertvolle Komponente zum Aufbau von Polyethern oder Polyestern. 3-Methyl-THF dient als Comonomer für die Herstellung von Polytetrahydrofuranen, die zu elastischen Fasern weiterverarbeitet werden (EP-A 343 985).

### Beispiele

Die in den Beispielen verwendeten Katalysatoren waren wie folgt zusammengesetzt (Angaben in Gew.-%) : Katalysator
- A: 67 % CoO
19,8 % CuO
7 % Mn₂O₃
3 % MoO₃
0,2 % NaO
3 % H₃PO₄
(nach EP-B 100 406, S.2, Zeile 63 - S.3, Zeile 11)
- B: 33 % CuO
38 % Cr₂O₃
9 % BaO
20 % H₂O
(nach DE-A 3 624 812, S.4, Zeile 35-41; Beispiele)
- C: 36,5 % CuO
1 % BaO
0,6 % Cr₂O₃
0,4 % ZnO
14,4 % MgO
28,5 % SiO₂
18,6 % H₂O
(nach DE-A 1 442 981, S. 2, 2. Absatz - S.3)
- D: 70 % CuO
25 % ZnO
5 % γ-Al₂O₃
(nach DE-A 1 542 632, S.2-S.4, Zeile 2)
- E: 40 % CuO
20 % ZnO
40 % γ-Al₂O₃
(nach DE-A 1 542 632, S.2-S.4, Zeile 2)
- F: 40 % CuO
40 % ZnO
19,9 % γ-Al₂O₃
0,1 % Na₂O
(nach DE-A 1 542 632, S.2 - S.4, Zeile 2)
- G: 56 % CuO
44 % Al₂O₃
(nach EP-A 44 444, S.8, Zeile 33 - S.11, Zeile 1)
- H: 35 % CuO
65 % γ-Al₂O₃
(nach EP-A 44 444, S.8, Zeile 33 - S. 11, Zeile 1)
- I: 1 % Ru
1,2 % Sn
1,3 % B
96,5 % γ-Al₂O₃
(nach Ind. Eng. Chem. Res., 28 (1989) 1110)

### Beispiele 1-8

In einem Rohrreaktor (Länge 20 cm, Durchmesser 16 mm) wurde an 30 ml Katalysator in Form von 2,5 bis 4 mm großem Splitt 3-Formyl-2-methyl-propionsäuremethylester in Form einer 20 gew.-%igen Lösung in Ethanol und 100 l (gemessen unter Normalbedingungen) Wasserstoff pro Stunde in absteigender Fahrweise bei 200 bar umgesetzt.

Der flüssige Reaktionsaustrag wurde gaschromatographisch untersucht.

Weitere Einzelheiten können der folgenden Tabelle 1 entnommen werden.

**Tabelle 1**

| Beispiel | Katalysator | Katalysatorbelastung [kg/kg · h] | Temperatur [°C] | Umsatz [%] | Ausbeute [%]* bezogen auf den Umsatz | |
|---|---|---|---|---|---|---|
| | | | | | Diol | 3-MTHF |
| 1 | A | 0,12 | 225 | 95 | 66 | 6 |
| 2 | B | 0,15 | 250 | 99 | 2 | 90 |
| 3 | C | 0,10 | 250 | 100 | 0 | 85 |
| 4 | D | 0,16 | 250 | 99 | 85 | 4 |
| 5 | E | 0,18 | 250 | 99 | 35 | 53 |
| 6 | F | 0,15 | 250 | 100 | 1 | 91 |
| 7 (ohne Ethanol) | G | 0,15 | 250 | 99 | 1 | 90 |
| 8** | H | - | 250 | 99 | 0 | 82 |
| Vergleichsbeispiel** | I | - | 250 | 100 | 1 | 69 |
| Diol = 2-Methyl-1,4-butandiol 3-MTHF = 3-Methyl-THF | | | | | | |

| | | | | | | |
|---|---|---|---|---|---|---|
| * Das Reaktionsgemisch enthielt weiterhin hauptsächlich 3-Methyl-γ-butyrolacton sowie nicht identifizierte Verbindungen. | | | | | | |
| ** Diskontinuierliche Umsetzung von 100 g 3-Formyl-2-methyl-propionsäuremethylester über 12 h bei 260 bar Wasserstoffdruck an 10g Katalysator. | | | | | | |

### Beispiele 9-11

In einem Rohrreaktor (Länge 20 cm, Durchmesser 16 mm) wurde Itaconsäure in Form einer 20 gew.-%igen Lösung in Ethanol sowie 100 l (gemessen unter Normalbedingungen) Wasserstoff pro Stunde bei 200 bar und einer Temperatur T an 2,5 - 4 mm Splitt-Katalysator umgesetzt. Der flüssige Reaktionsaustrag wurde gaschromatographisch untersucht.

Weitere Einzelheiten können Tabelle 2 entnommen werden.

**Tabelle 2**

| Beispiel | Katalysator | Menge an Katalysator [g] | Katalysator-Belastung [kg/kg · h] | Temperatur [°C] | Umsatz [%] | Ausbeute [%] * bezogen auf den Umsatz | |
|---|---|---|---|---|---|---|---|
| | | | | | | Diol | 3-MTHF |
| 9 | A | 29 | 0,10 | 225 | 95 | 66 | 6 |
| 10 | F | 23 | 0,10 | 250 | 97 | 1 | 90 |
| 11 | G | 19 | 0,20 | 250 | 100 | 1 | 91 |
| Diol = 2-Methyl-1,4-butandiol 3-MTHF = 3-Methyl-THF | | | | | | | |

| | | | | | | | |
|---|---|---|---|---|---|---|---|
| * s. Tabelle 1 | | | | | | | |

## Patentansprüche

1. Verfahren zur Herstellung von 2-Methyl-1,4-butandiol und 3-Methyltetrahydrofuran, dadurch gekennzeichnet, daß man Verbindungen der allgemeinen Formeln I und II 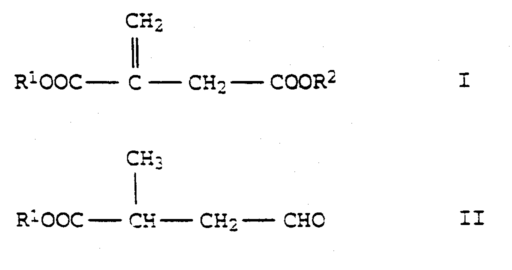 in denen R¹ und R² für Wasserstoff oder eine C₁-C₈-Alkylgruppe stehen und die Formylgruppe von II auch als Acetal mit C₁-C₈-Alkanolen vorliegen kann, in Gegenwart von Kupfer oder von Kupferverbindungen bei einem Druck von 50 bis 300 bar mit Wasserstoff umsetzt.

2. Verfahren nach Anspruch 1, dadurch gekennzeichnet, daß man Itaconsäure oder 3-Formyl-2-methyl-propionsäuremethylester umsetzt.

## Claims

1. A process for the preparation of 2-methyl-1,4-butanediol and 3-methyltetrahydrofuran, wherein a compound of the formula I or II 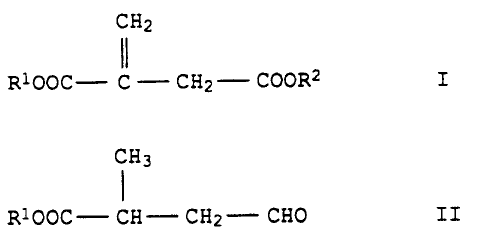 where R¹ and R² are each hydrogen or C₁-C₈-alkyl and the formyl group of II may also be present as acetal with a C₁-C₈-alkanol, is reacted with hydrogen in the presence of copper or a copper compound at from 50 to 300 bar.

2. A process as claimed in claim 1, wherein itaconic acid or methyl 3-formyl-2-methylpropionate is converted.

## Revendications

1. Procédé de préparation du 2-méthyl-1,4-butanediol et du 3-méthyltétrahydrofuranne, caractérisé en ce que l'on fait réagir des composés des formules générales I et II 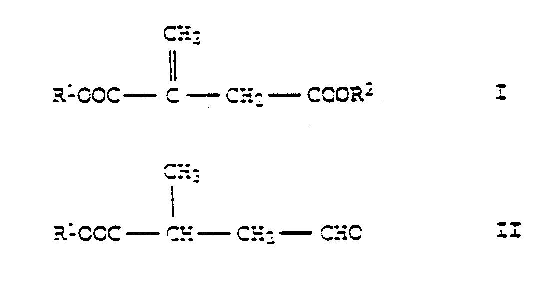 dans lesquelles R¹ et R² représentent chacun un atome d'hydrogène ou un radical alkyle en C₁ à C₈ et le groupe formyle du composé II peut également se présenter sous la forme d'acétal avec des alcanols en C₁ à C₈, en présence de cuivre ou de composés du cuivre, avec l'hydrogène, sous une pression de 50 à 300 bars.

2. Procédé suivant la revendication 1, caractérisé en ce que l'on fait réagir l'acide itaconique ou le 3-formyl-2-méthylpropionate de méthyle.
